# EUROPEAN PATENT APPLICATION

(11) **EP 4 809 926 A1**
(43) Date of publication of application: **23.09.2026**
(21) Application number: 24891092.9
(22) Date of filing: 25.09.2024
(51) Int. Cl.: A61B 5/397

(54) **INFORMATION PROCESSING DEVICE AND INFORMATION PROCESSING SYSTEM**

(30) Priority: 15.11.2023 JP 2023194584
(71) Applicant: Sony Group Corporation, Tokyo 108-0075 (JP)
(72) Inventor: KITAKAMI, Yukinojo, Tokyo 108-0075 (JP); ISHIKAWA, Takanori, Tokyo 108-0075 (JP); YOSHIKAWA, Kiyoshi, Tokyo 108-0075 (JP); MATSUKAWA, Ryotaro, Tokyo 108-0075 (JP)
(74) Representative: D Young & Co LLP
(86) International application number: PCT/JP2024/034053
(87) International publication number: WO 2025/105050

(57) **Abstract**

An information processing device according to an embodiment of the present disclosure includes: a measurer that includes an electrode mountable on a living body and is configured to output a first signal based on a potential of the electrode; a first processor configured to generate a second signal related to a period in which a myoelectric potential of the living body changes; and a determiner configured to determine a mounting state of the electrode on the basis of the first signal and the second signal.

## Description

### Technical Field

The present disclosure relates to an information processing device and an information processing system.

### Background Art

A brainwave measurement system has been proposed that includes electrodes for brainwave measurement, and determines whether a mounting state of the electrode is favorable or poor by using an amount of mixed alternating-current noise (PTL 1).

### Citation List

### Patent Literature

PTL 1: International Publication No. WO 2011/158481

### Summary of the Invention

In a device that measures a potential, it is desirable to be able to determine a mounting state of an electrode.

It is desirable to provide an information processing device that makes it possible to suitably determine a mounting state.

An information processing device according to an embodiment of the present disclosure includes: a measurer that includes an electrode mountable on a living body and is configured to output a first signal based on a potential of the electrode; a first processor configured to generate a second signal related to a period in which a myoelectric potential of the living body changes; and a determiner configured to determine a mounting state of the electrode on the basis of the first signal and the second signal.

An information processing system according to an embodiment of the present disclosure includes: a measurer that includes an electrode mountable on a living body and is configured to output a first signal based on a potential of the electrode; and an information processing device including a first processor configured to generate a second signal related to a period in which a myoelectric potential of the living body changes, and a determiner configured to determine a mounting state of the electrode on the basis of the first signal and the second signal.

### Brief Description of Drawings

[FIG. 1] FIG. 1 is a diagram illustrating an example of a schematic configuration of an information processing device according to an embodiment of the present disclosure.
[FIG. 2] FIG. 2 is a diagram for describing a configuration example of a measurer according to the embodiment of the present disclosure.
[FIG. 3] FIG. 3 is a diagram for describing a configuration example of the information processing device according to the embodiment of the present disclosure.
[FIG. 4A] FIG. 4A is a diagram illustrating an example of a measurement signal that is obtained by the information processing device according to the embodiment of the present disclosure.
[FIG. 4B] FIG. 4B is a diagram illustrating an example of the measurement signal that is obtained by the information processing device according to the embodiment of the present disclosure.
[FIG. 5] FIG.5 is a diagram for describing an example of a determination process that is performed by the information processing device according to the embodiment of the present disclosure.
[FIG. 6A] FIG. 6A is a diagram for describing an example of a calibration task that is set by the information processing device according to the embodiment of the present disclosure.
[FIG. 6B] FIG. 6B is a diagram for describing an example of the calibration task that is set by the information processing device according to the embodiment of the present disclosure.
[FIG. 6C] FIG. 6C is a diagram for describing an example of the calibration task that is set by the information processing device according to the embodiment of the present disclosure.
[FIG. 7A] FIG. 7A is a diagram for describing an example of a method of presenting the calibration task by the information processing device according to the embodiment of the present disclosure.
[FIG. 7B] FIG. 7B is a diagram for describing an example of the method of presenting the calibration task by the information processing device according to the embodiment of the present disclosure.
[FIG. 8] FIG. 8 is a flowchart illustrating an operation example of the information processing device according to the embodiment of the present disclosure.
[FIG. 9] FIG. 9 is a diagram for describing a configuration example of the information processing device according to the embodiment of the present disclosure.
[FIG. 10] FIG. 10 is a diagram for describing a configuration example of the information processing device according to the embodiment of the present disclosure.
[FIG. 11] FIG. 11 is a diagram for describing a configuration example of the information processing device according to the embodiment of the present disclosure.
[FIG. 12] FIG. 12 is a diagram for describing a configuration example of an information processing device according to a modification example of the present disclosure.
[FIG. 13] FIG. 13 is a flowchart illustrating an operation example of the information processing device according to the modification example of the present disclosure.

### Modes for Carrying Out the Invention

Some embodiments of the present disclosure are described below in detail with reference to the drawings. It is to be noted that description is given in the following order.
0. Background
1. Embodiment
2. Modification Examples

### <0. Background>

In a case of measuring a brainwave, for example, as biological information, electrodes are installed on a skin surface of a head, an ear, or the like, and a potential change caused by a neural activity of a brain is measured. It is important that the electrodes be securely mounted in order to accurately measure the potential change caused by the neural activity of the brain. In a poor mounting state of the electrode on the skin, the potential can fail to be measured in the first place, or a measurement signal can be contaminated by various electromagnetic noises present around a body. This can prevent accurate measurement of the potential change caused by the neural activity of the brain, which is to be measured. Accordingly, it is desirable to make it possible to appropriately determine a contact state of the electrode with the skin.

### <1. Embodiment>

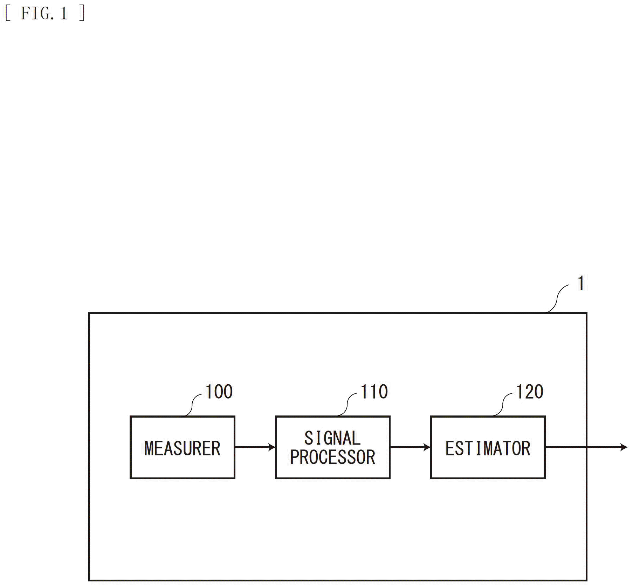

FIG. 1 is a diagram illustrating an example of a schematic configuration of an information processing device according to an embodiment of the present disclosure. An information processing device 1 is a device configured to acquire a signal related to a living body (hereinafter referred to as a biological signal). The information processing device 1 includes a measurer 100 including a plurality of electrodes configured to be in contact with the living body, and is configured to detect the biological signal. The biological signal may also be referred to as a signal based on a bioelectric potential, i.e., a bioelectric potential signal.

The information processing device 1 is configured to execute biological measurement of a brainwave (EEG: Electroencephalography), a cardiac signal (ECG: Electrocardiogram), or the like. The information processing device 1 is, for example, a device configured to measure the bioelectric potential, and may be referred to as a bioelectric potential measurement device (or a bioelectric potential measurement circuit). Further, the information processing device 1 is a device configured to acquire the biological signal, and may be referred to as a biological signal acquisition device.

The biological signal is, for example, a signal related to a potential (a voltage) generated as a result of activity of the living body. Examples of the biological signal include a brainwave that is a signal associated with cerebral activity, a myoelectric signal associated with muscular activity, and a cardiac signal associated with cardiac activity. The biological signal is a signal related to the brainwave, a myoelectric potential, a cardiac potential, a pulse wave, or the like.

The information processing device 1 detects the biological signal, such as a signal related to the brainwave (a brainwave signal). The detected brainwave signal serves as, for example, an electrical signal corresponding to an activity state of a user's brain. The information processing device 1 may measure the potential (perform bioelectric potential measurement) with use of the electrode mounted on a body surface of the user, to measure the brainwave, the myoelectric signal, or the like. The information processing device 1 acquires the biological signal based on the bioelectric potential, making it possible to check a state of the living body.

The information processing device 1 is applicable to, for example, an electronic apparatus to be worn by the user. The information processing device 1 may be applied to the electronic apparatus that is wearable on a body including an ear, a head, a face, a neck, a hand, a wrist, an arm, a foot, a chest, etc. The information processing device 1 may be implemented as a device to be mounted on wearable devices including, for example, earphones, headphones, and eyewear.

The information processing device 1 includes the measurer 100 and a signal processor 110, as in the example illustrated in FIG. 1. The information processing device 1 may also include an estimator 120. The estimator 120 may be provided in the signal processor 110 or may be provided separately from the signal processor 110. The information processing device 1 (or the measurer 100) may be configured, for example, as a sensor (a biological sensor) that is configured to acquire the biological signal.

The measurer 100 is a measurement circuit and is configured to generate the biological signal. The measurer 100 includes the plurality of electrodes for detecting the potential, which will be described later. The measurer 100 (the measurement circuit) is, for example, configured to execute measurement of the brainwave, the myoelectric signal, or the like. The measurer 100 may acquire the biological signal of the user and output the biological signal to the signal processor 110.

The signal processor 110 is a signal processing circuit and is configured to execute signal processing (information processing). The signal processor 110 includes, for example, a processor and memories (ROM, RAM and the like), and is configured to perform various kinds of signal processing. The signal processor 110 may read and execute a program incorporated therein, and perform the signal processing (the information processing).

The signal processor 110 is configured to execute the signal processing on a signal to be inputted. The signal processor 110 (the signal processing circuit) includes, for example, a circuit that performs various kinds of signal processing on the biological signal. The signal processor 110 may perform various kinds of signal processing including, for example, a process of reducing noises, a frequency-analysis process, and a normalization process on the biological signal to be outputted from the measurer 100.

The estimator 120 is configured to execute a process of estimating a state of the living body with use of the biological signal. The estimator 120 includes, for example, a processor, a memory, and the like. The estimator 120 may perform, for example, a process of calculating a feature quantity using the biological signal after being subjected to a noise-reduction process. The estimator 120 extracts the feature quantity by performing, for example, a Fast Fourier Transform (FFT) process or a wavelet analysis process on the biological signal. Examples of the feature quantity include a α-wave component, a β-wave component, and a γ-wave component that are included in the biological signal.

The estimator 120 performs a process of estimating the state of the living body on the basis of a result of the calculation of the feature quantity. The estimator 120 estimates the state such as whether or not the living body is relaxed or whether or not the living body is concentrating, for example, on the basis of the α-wave component and the β-wave component. The estimator 120 may be configured to determine the state of the living body.

As another example, the estimator 120 may determine whether or not the living body is in a sleeping state with use of the biological signal related to the brainwave. Further, for example, the estimator 120 may estimate a heart rate with use of the biological signal related to the cardiac signal. The estimator 120 may estimate an emotion of the living body by analyzing the biological signal. Thus analyzing the biological signal makes it possible to check the state of the living body.

The information processing device 1 may be configured to acquire information (context information) related to a state or a situation of the user, and execute a process of estimating the state of the living body, a process of proposing (presenting) an action to the user, and the like. The context information includes, for example, information related to an action (a motion) of the user, information related to a position of the user, information related to a time, and information related to a sound.

The information processing device 1 (or the measurer 100) may include, for example, as a sensor unit, various sensors configured to acquire the context information, such as an acceleration sensor, a gyro sensor (an angular velocity sensor), a GPS sensor, and a sound sensor. The sensor unit may be provided in the information processing device 1 or may be provided separately from the information processing device 1.

The estimator 120 is configured to generate information indicating the state of the living body (hereinafter referred to as state information). The estimator 120 may generate and output the state information as an estimation result. The state information includes, for example, information indicating whether or not the living body is in a relaxed state, information indicating whether or not the living body is in a sleeping state, information indicating a psychological state such as an emotion of the living body, and information indicating a heart rate.

The state information may be used, for example, for displaying an image indicating the state of the living body, outputting a sound indicating the state of the living body, and the like. The information processing device 1 may include, for example, a display unit configured to display an image based on the state information. The display unit is, for example, an organic EL display, a liquid crystal display, or the like. The display unit may include a touch panel.

The information processing device 1 may include a sound output unit, such as a speaker, that is configured to output a sound (a music piece (BGM), a sound effect, or the like) based on the state information. The information processing device 1 may be configured to apply a vibration generated by, for example, a vibration function to the user on the basis of the state information.

The information processing device 1 includes, for example, a presentation unit (the display unit, the sound output unit, or the like) configured to present (provide) an image, a sound, a vibration, or the like to the user. The information processing device 1 may be configured to decide (determine) an action recommended to the user with use of the biological information and the context information. The information processing device 1 may cause the display unit to display an image that prompts the recommended action, and may cause the sound output unit to output a voice message that prompts the recommended action.

It is to be noted that the signal processor 110, the estimator 120, or both may be provided in an external device that is provided outside the information processing device 1. Examples of the external device include an electronic apparatus that is a terminal device (a terminal) to be used by the user, a server (for example, a cloud server), and the like. Examples of the electronic apparatus include a smartphone, a tablet terminal, a wearable terminal, a computer, and another information processing device.

A combination of the information processing device 1 and the external device may also be referred to as an information processing device. The information processing device 1 and the external device may transmit and receive a signal (information) via wireless or wired communication. For example, the information processing device 1 is configured to be coupled to a network and to communicate with the external device. A combination of the information processing device 1 and the external device coupled to each other via the network may also be referred to as an information processing device or an information processing system.

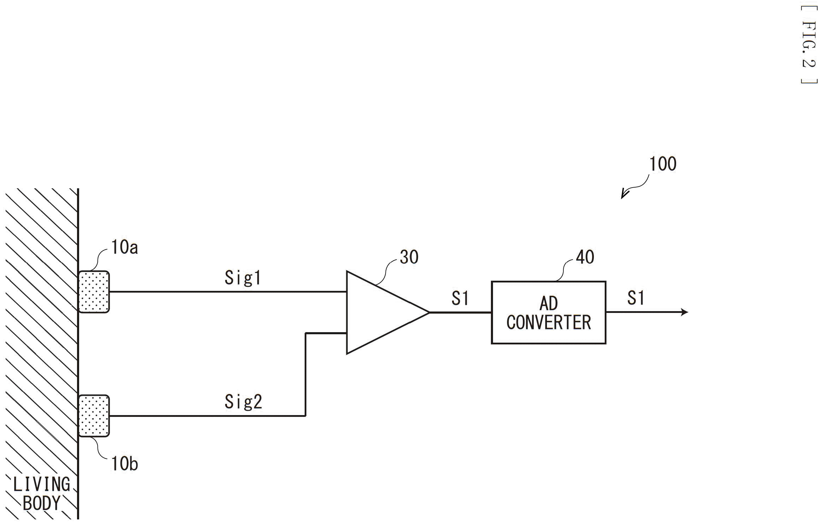

FIG. 2 is a diagram for describing a configuration example of the measurer according to the embodiment. The measurer 100 includes an electrode to be used for detecting a potential, and is configured to measure the bioelectric potential. The measurer 100 includes, for example, a plurality of electrodes 10 (a signal electrode 10a and a reference electrode 10b in FIG. 2), an amplifier circuit 30, and an AD converter circuit 40.

The signal electrode 10a and the reference electrode 10b are each mountable on the living body. The signal electrode 10a and the reference electrode 10b are provided away from each other, and come into contact with respective positions that are different from each other. The signal electrode 10a is configured to be disposed at any position from which the biological signal is to be acquired. The reference electrode 10b is configured to be disposed at any position including the vicinity of the signal electrode 10a.

The measurer 100 detects a potential (a voltage) of a surface of the living body with use of the signal electrode 10a and the reference electrode 10b. For example, electricity generated in the living body may cause a potential difference between the signal electrode 10a and the reference electrode 10b that are brought into contact with a skin of the living body.

The signal electrode 10a and the reference electrode 10b each include an electrically conductive material. The signal electrode 10a and the reference electrode 10b each include, for example, silver/silver chloride (Ag/AgCl), aluminum (Al), copper (Cu), gold (Au), or the like. The signal electrode 10a and the reference electrode 10b may each include, for example, an electrically conductive and elastic resin material. It is to be noted that a shape of each of the signal electrode 10a and the reference electrode 10b is not particularly limited, and may be a circular shape, an oval shape, a polygonal shape, or another shape.

The signal electrode 10a comes into contact with a measurement site (a point to be measured) in actual use, and a potential of the contact site is applied, as in the example schematically illustrated in FIG. 2. The signal electrode 10a is disposed, for example, directly on an activity region, of the living body, in which the biological signal is to be measured. The signal electrode 10a is electrically coupled to the amplifier circuit 30, for example, and supplies a signal Sig1 to the amplifier circuit 30. The signal Sig1 is a signal corresponding to the potential (the bioelectric potential) of the contact site of the living body. The signal Sig1 is a biological signal obtained by the signal electrode 10a.

The reference electrode 10b comes into contact with the living body at a position that differs from the position of the signal electrode 10a in actual use, and a potential of the contact site is applied, as in the example illustrated in FIG. 2. The reference electrode 10b may be disposed at any position around the signal electrode 10a, for example. The reference electrode 10b is electrically coupled to the amplifier circuit 30, for example, and supplies a signal Sig2 to the amplifier circuit 30. The signal Sig2 corresponds to the potential of the contact site of the living body. The signal Sig2 is a biological signal obtained by the reference electrode 10b.

The amplifier circuit 30 is configured to amplify a signal to be inputted. The amplifier circuit 30 includes, for example, a differential amplifier circuit (a differential amplifier) configured to amplify a signal. The amplifier circuit 30 (a differential amplifying unit) generates a measurement signal S1 on the basis of, for example, a signal obtained by the signal electrode 10a and a signal obtained by the reference electrode 10b. The measurement signal S1 is a biological signal based on a potential of the signal electrode 10a and a potential of the reference electrode 10b.

In the example illustrated in FIG. 2, the signal Sig1 is inputted to the amplifier circuit 30 by the signal electrode 10a. Further, the signal Sig2 is inputted to the amplifier circuit 30 by the reference electrode 10b. The amplifier circuit 30 generates, for example, the measurement signal S1 based on a difference between the signal Sig1 and the signal Sig2. The amplifier circuit 30 may generate the measurement signal S1 corresponding to a difference between a potential of the signal Sig1 and a potential of the signal Sig2, and output the generated measurement signal S1 to the AD converter circuit 40.

The AD converter circuit 40 is configured to convert an analog signal to be inputted into a digital signal. The AD converter circuit 40 is an ADC (Analog to Digital Converter). The measurement signal S1 is inputted to the AD converter circuit 40 from the amplifier circuit 30, for example. The AD converter circuit 40 performs an AD conversion process on the measurement signal S1 as the analog signal inputted from the amplifier circuit 30.

The AD converter circuit 40 (an AD converter) converts the measurement signal S1 outputted by the amplifier circuit 30 into the digital signal. The AD converter circuit 40 may output the measurement signal S1 converted into the digital signal to the signal processor 110 (see FIG. 1). The signal processor 110 acquires the measurement signal S1 as the biological signal from the measurer 100, and performs various kinds of signal processing on the measurement signal S1. The estimator 120 may perform, for example, a process of generating the state information with use of the measurement signal S1.

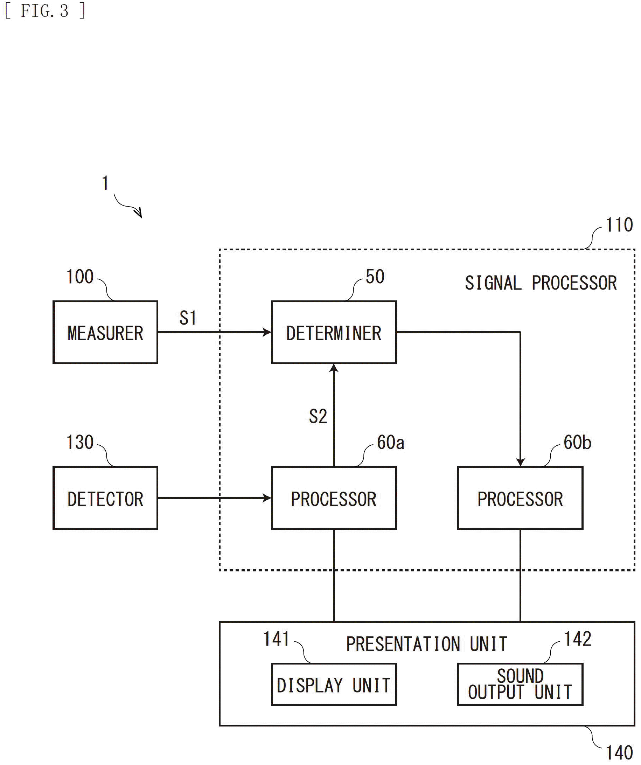

FIG. 3 is a diagram for describing a configuration example of the information processing device according to the embodiment. The information processing device 1 includes the signal processor 110 as in the example illustrated in FIG. 3. The information processing device 1 may also include the measurer 100 described above, a detector 130, and a presentation unit 140.

The measurer 100 includes the electrodes 10 (e.g., the signal electrode 10a and the reference electrode 10b) mountable on the living body, and is configured to acquire the measurement signal S1 based on the potentials of the electrodes 10, as described above. The measurer 100 generates the measurement signal S1, and outputs the measurement signal S1 to the signal processor 110.

The detector 130 is configured to detect mounting of the electrode 10 on the living body. The detector 130 is configured to, for example, detect that the electrode 10 is mounted on the living body, in response to a signal (an operation signal) based on an operation on an unillustrated operation member by the user.

The detector 130 may be configured to detect the mounting of the electrode 10 on the living body, on the basis of an output signal of a sensor configured to detect contact (or proximity) of the electrode 10 with the living body. Examples of the sensor include an acceleration sensor, an optical sensor, an ultrasonic sensor, a temperature sensor, and a sound sensor (a microphone).

The detector 130 may include, for example, the operation member (an operation button or switch, a touch panel, etc.) and the sensor that detects the contact (or proximity) of the electrode 10 (the acceleration sensor, the optical sensor, etc.). The signal processor 110 may include the detector 130. The detector 130 may generate a signal (a detection signal) indicating that the electrode 10 is mounted on the living body, and output the signal to a processor 60a of the signal processor 110.

The presentation unit 140 is configured to present images and sounds to the user. In the example illustrated in FIG. 3, the presentation unit 140 includes a display unit 141 configured to display an image and a sound output unit 142 configured to output a sound. The display unit 141 is configured to display an image on the basis of image data. The display unit 141 is an organic EL display, a liquid crystal display, or the like. It is to be noted that the display unit 141 may include a touch panel.

The sound output unit 142 is configured to output a sound based on audio data. The sound output unit 142 is a speaker or the like, and may also be referred to as a converter that converts the audio data as an electric signal into the sound (a sound wave). The sound output unit 142 may output the sound corresponding to the audio data. It is to be noted that a part or all of the display unit 141 and the sound output unit 142 may be integrally configured.

The signal processor 110 includes, for example, a determiner 50, the processor 60a, and a processor 60b. The determiner 50 is configured to determine a mounting state of the electrode 10 on the basis of a signal outputted from the measurer 100. The determiner 50 may determine whether or not the mounting state (a contact state) of the electrode 10 on the living body is favorable, by using a signal component of the myoelectric potential included in the measurement signal S1 that is measured by the measurer 100.

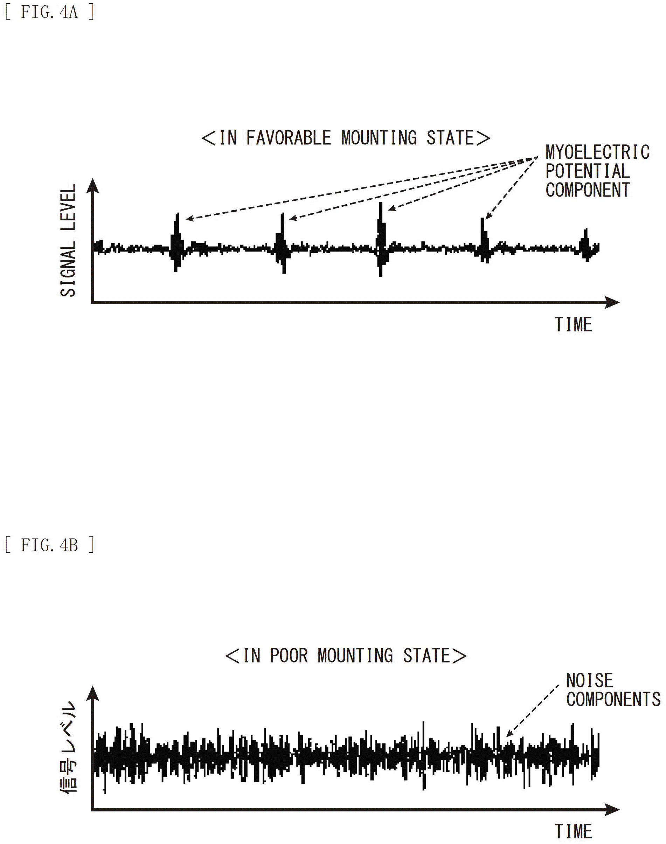

FIG. 4A is a diagram illustrating an example of the measurement signal in a favorable mounting state of the electrode 10. Further, FIG. 4B is a diagram illustrating an example of the measurement signal in a poor mounting state of the electrode 10. In FIGs. 4A and 4B, the vertical axis represents a signal level of the measurement signal S1, and the horizontal axis represents a time.

In a favorable mounting state of the electrode 10, a change in the myoelectric signal caused by a muscle of the living body is observed, as in the example illustrated in FIG. 4A. A signal value (an amplitude) of the measurement signal S1 increases and decreases in response to tensing and relaxing. For example, in a state in which the electrode 10 is mounted on the ear or around the ear, the amplitude (the signal level) of the measurement signal S1 increases upon tensing of a facial muscle, and the amplitude of the measurement signal S1 decreases upon relaxing of the facial muscle. In a poor mounting state of the electrode 10, various noises (e.g., electromagnetic noises) tend to be mixed into the measurement signal S1, as in the example illustrated in FIG. 4B.

The determiner 50 is configured to determine the mounting state of the electrode 10 on the basis of, for example, the amplitude of the measurement signal S1 in a period in which the myoelectric potential of the living body changes. The determiner 50 may determine the mounting state of the electrode 10 on the basis of a distribution of values of the measurement signal S1 in the period in which the myoelectric potential of the living body changes. The determiner 50 may determine (estimate) whether the contact state of the electrode 10 is favorable or poor, by grasping the component of the myoelectric potential mixed into the measurement signal S1.

The processor 60a is configured to generate a signal (referred to as a timing signal) related to the period in which the myoelectric potential of the living body changes. The processor 60a is configured to, for example, generate a timing signal S2 related to a period in which the myoelectric potential included in the measurement signal S1 changes (transitions), and output the timing signal S2 to the determiner 50.

The processor 60a generates the timing signal S2 indicating, for example, a period in which the user performs an action involving a change in the myoelectric potential. The period in which the action involving a change in the myoelectric potential is performed may be automatically set by the information processing device 1 or may be set by the user. The processor 60a may generate, for example, as the timing signal S2, a signal related to a start timing and an end timing of the action involving a change in the myoelectric potential, and output the signal to the determiner 50.

The determiner 50 is configured to determine the mounting state of the electrode 10 on the living body, on the basis of the measurement signal S1 that is inputted from the measurer 100 and the timing signal S2 that is inputted from the processor 60a. The determiner 50 is configured to, for example, determine the mounting state of the electrode 10 on the basis of the measurement signal S1 in the period in which the myoelectric potential of the living body changes as indicated by the timing signal S2. For example, the determiner 50 may determine the mounting state by using the values of the measurement signal S1, the distribution (a shape) of the values of the measurement signal S1, or the like in the period in which the myoelectric potential changes.

The determiner 50 is configured to generate a signal (referred to as a determination signal) related to a result of the determination of the mounting state. The determiner 50 may generate the determination signal related to the mounting state of the electrode 10 and output the determination signal to the processor 60b. The determination signal is, for example, a signal indicating the mounting state of the electrode 10, such as a signal indicating a favorable mounting state (contact state) of the electrode 10 or a signal indicating a poor mounting state of the electrode 10.

The processor 60b is configured to execute a process of presenting an image, a sound, or both based on the result of the determination by the determiner 50. The processor 60b is configured to, for example, control the presentation unit 140 in response to the determination signal outputted from the determiner 50. For example, the processor 60b controls the presentation unit 140 to present an image or a sound indicating the mounting state of the electrode 10 to the user.

Further, the processor 60b may control the presentation unit 140 to output an image, a sound, or the like that prompts re-mounting of the electrode 10 (or the information processing device 1). It is to be noted that a part or all of the processor 60a and the processor 60b described above may be integrally configured.

The processor 60a is configured to execute a process of prompting an action involving a change in the myoelectric potential. The processor 60a is configured to execute, for example, a process of presenting an image, a sound, or both that prompt an action (referred to as a calibration task) involving a change in the myoelectric potential. The processor 60a may be configured to execute a process of prompting the user to perform the calibration task in a case where, for example, the mounting of the electrode 10 is detected by the detector 130.

In the example illustrated in FIG. 3, the processor 60a may control the presentation unit 140 to present an image, a sound, or the like that prompts the calibration task to the user. The calibration task is, for example, an action (a process) for changing the myoelectric potential of the living body, and is an action of tensing and relaxing the muscle of the face or the neck in order. The processor 60a is configured to, for example, control the presentation unit 140 to prompt the user to perform the calibration task that includes tensing the muscle at least once and relaxing the muscle at least once.

The processor 60a may perform a process of causing the display unit 141 to display an image indicating the calibration task. The processor 60a controls the display unit 141, for example, and causes the display unit 141 to display an image indicating a timing of tensing the muscle and a timing of relaxing the muscle. The processor 60a may also perform a process of causing the sound output unit 142 to output a voice message, a sound effect (a beep sound), or the like that orders (requests) tensing and relaxing of the muscle.

The processor 60a is configured to generate a signal related to a period of the calibration task as the timing signal S2. The processor 60a generates, for example, the timing signal S2 related to a start timing and an end timing of the calibration task, and outputs the timing signal S2 to the determiner 50. The timing signal S2 may also be referred to as a signal indicating a time during which the user's action to change the myoelectric potential is performed.

The determiner 50 is configured to determine the mounting state of the electrode 10 on the basis of the measurement signal S1 in the period of the calibration task indicated by the timing signal S2. The determiner 50 may determine the mounting state with use of, for example, the values of the measurement signal S1, the distribution of the values of the measurement signal S1, or the like in the period in which the calibration task is executed. The information processing device 1 makes it possible to accurately perform the determination of the mounting state using the measurement signal S1 by causing a change in the myoelectric potential mixed into the measurement signal S1 through the calibration task.

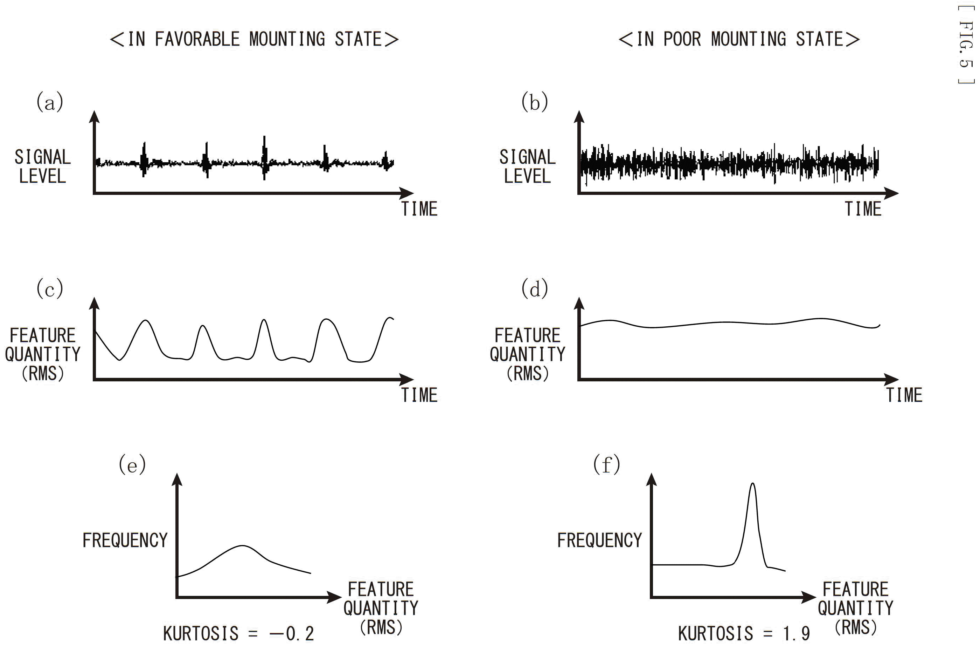

FIG. 5 is a diagram for describing an example of a determination process that is performed by the information processing device according to the embodiment. Part (a) of FIG. 5 illustrates an example of the measurement signal S1 during the calibration task in a favorable mounting state of the electrode 10. Part (b) of FIG. 5 illustrates an example of the measurement signal S1 during the calibration task in a poor mounting state of the electrode 10.

The determiner 50 is configured to, for example, execute a process of calculating the feature quantity with use of the measurement signal S1. The determiner 50 may calculate, for example, an amplitude intensity as the feature quantity that changes (increases or decreases) in response to the myoelectric signal. The determiner 50 is configured to calculate, for example, a square root (RMS: root mean square) as the amplitude intensity. A value of the RMS at a given time may be expressed by, for example, the following expression (1).
[Math. 1] $RMS = \sqrt{\frac{1}{N} {\sum }_{i = 1}^{N} {x}_{i}^{2}}$

In the expression (1), x is the value of the measurement signal S1 at the given time. N is the number of samples in a given interval. In a favorable mounting state, the feature quantity (the RMS value ) temporally changes in response to tensing and relaxing of the muscle, as in the example illustrated in part (c) of FIG. 5. In contrast, in a poor mounting state, for example, the feature quantity is substantially constant, as in the example illustrated in part (d) of FIG. 5.

Parts (e) and (f) of FIG. 5 each illustrate the distribution of the feature quantity, with the horizontal axis representing the feature quantity (the RMS value) and the vertical axis representing a frequency. In a favorable mounting state, because the feature quantity temporally changes, the distribution of the feature quantity is relatively flat as in the example illustrated in part (e) of FIG. 5. In contrast, in a poor mounting state, as a result of the substantially constant feature quantity, the distribution of the feature quantity is a distribution having a peak at a given value, as in the example illustrated in part (f) of FIG. 5.

The determiner 50 calculates, for example, a kurtosis as an index representing how pointed a frequency distribution is, as illustrated in parts (e) and (f) of FIG. 5. For example, the kurtosis is -0.2 in the example illustrated in part (e) of FIG. 5, and the kurtosis is 1.9 in the example illustrated in part (f) of FIG. 5. The determiner 50 may determine the mounting state of the electrode 10 by comparing the calculated kurtosis with a threshold. It is to be noted that the threshold to be used for determining the favorable or poor mounting state may be adjusted (changed) depending on an arrangement of the electrodes 10, a manner of applying tension and relaxation in the calibration task, or the like.

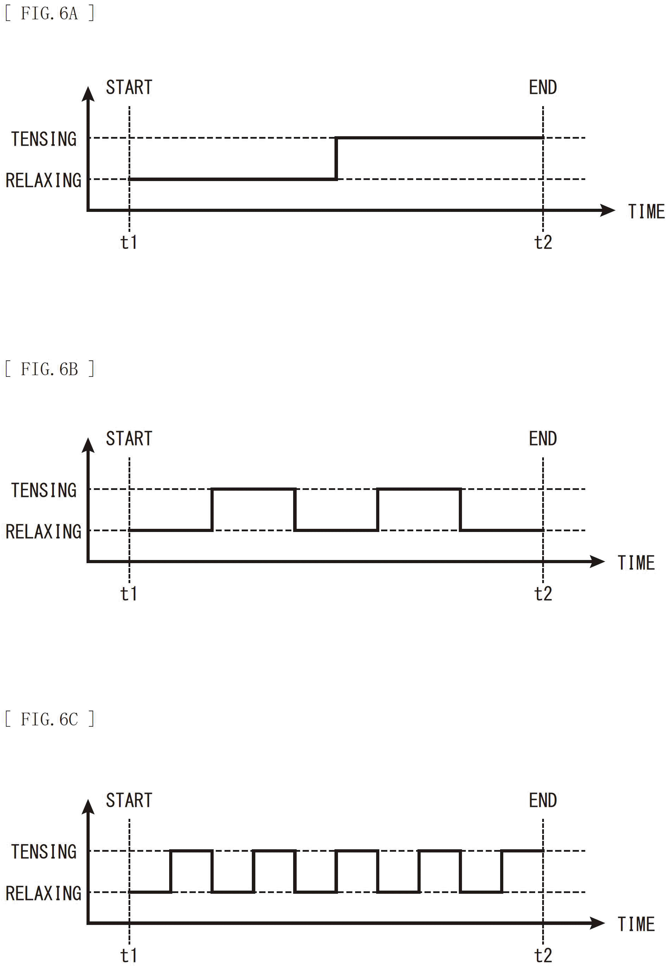

FIGs. 6A to 6C are each a diagram for describing an example of the calibration task that is set by the information processing device according to the embodiment. The calibration task includes, for example, tensing the muscle at least once and relaxing the muscle at least once, as schematically illustrated in FIG. 6A. The calibration task may be an action of tensing the muscle a plurality of times and relaxing the muscle a plurality of times, as in the example illustrated in FIG. 6B. Increasing the number of times of tensing and relaxing makes it possible to improve the accuracy of the determination process of the mounting state.

Further, the calibration task may be set to apply tension and apply relaxation for substantially the same time. For example, in the example illustrated in FIG. 6C, a ratio of the time of tensing and a ratio of the time of relaxing are substantially equal to each other. In a case where the time ratios of tensing and relaxing during calibration are equal to each other, a favorable mounting state makes the distribution of the feature quantity (e.g. the RMS value) flat and results in a lower kurtosis. This makes it possible to accurately determine the mounting state.

In the examples illustrated in FIGs. 6A to 6C, the processor 60a may generate, for example, the timing signal S2 related to the start timing (a time t1) and the end timing (a time t2) of the calibration task, and output the timing signal S2 to the determiner 50. It is possible for the determiner 50 to appropriately determine the mounting state of the electrode 10, by analyzing the measurement signal S1 in a period from the time t1 as the start timing of the calibration task to the time t2 as the end timing of the calibration task, on the basis of the timing signal S2 outputted by the processor 60a.

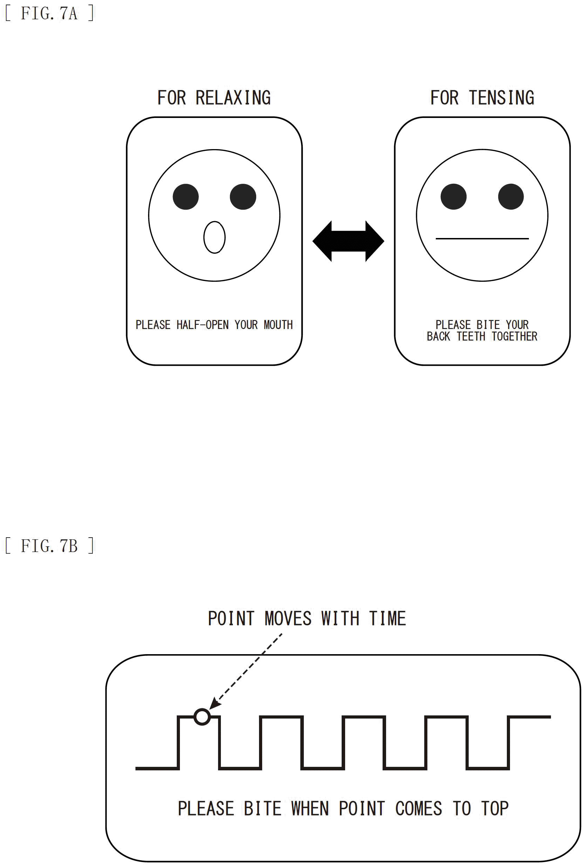

FIGs. 7A and 7B are each a diagram for describing an example of a method of presenting the calibration task by the information processing device according to the embodiment. For example, the information processing device 1 may execute a presentation that prompts the action at the timings of tensing and relaxing, as in the example illustrated in FIG. 7A. The processor 60a of the information processing device 1 may cause the presentation unit 140 to display images illustrated in FIG. 7A, or may cause a display of an electronic apparatus provided outside the information processing device 1 to display the images illustrated in FIG. 7A.

As illustrated in FIG. 7B, the processor 60a of the information processing device 1 may present the calibration task including the action of tensing and relaxing by an image indicating a time-series pattern and movement of a point. Using a point (a circle), another mark, or the like that moves with time, as in the example illustrated in FIG. 7B, makes it possible to present which action is be performed in a manner easily understandable to the user.

The information processing device 1 may perform a presentation of the calibration task using voice. For example, the sound output unit 142 may output a voice such as "please half-open your mouth" or "please relax" for relaxing. Further, the sound output unit 142 may output a voice message such as "please bite your back teeth together" or "please bite your back teeth" for tensing.

The information processing device 1 may present repetition of tensing and relaxing at intervals of a short time (e.g., a time of 1 second or less) by performing a presentation using a beep sound. For example, the information processing device 1 may present "please bite your back teeth while sound is heard" before the start of the calibration task, and present a beep sound at the timing of tensing.

The processor 60b may cause the presentation unit 140 or the electronic apparatus provided outside the information processing device 1 to output an image or a sound corresponding to the result of the determination by the determiner 50. For example, the processor 60b may control the presentation unit 140 to present "mounting is completed", "device is to be re-mounted because of poor mounting state", or the like.

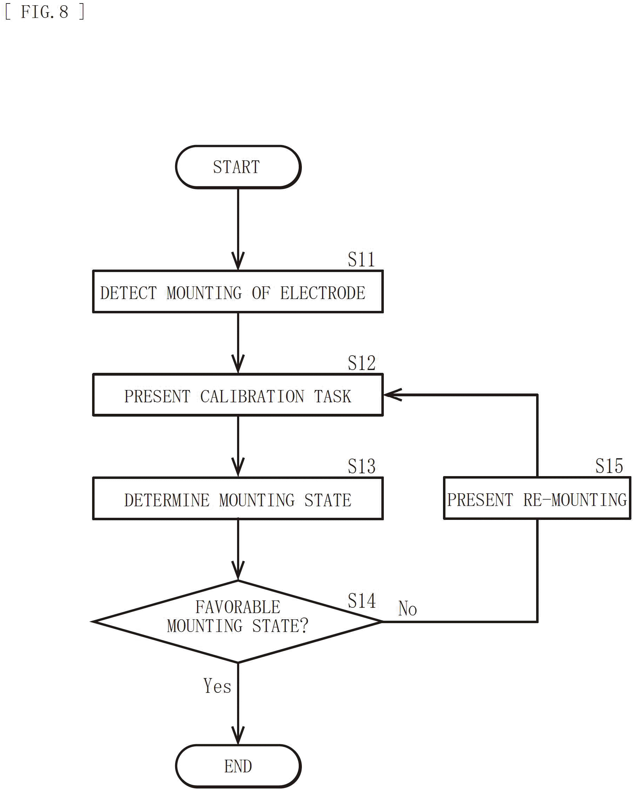

FIG. 8 is a flowchart illustrating an operation example of the information processing device according to the embodiment. An operation example of the information processing device 1 will be described with reference to the flowchart of FIG. 8. For example, the flowchart of FIG. 8 is started after an application of the information processing device 1 is started up.

In step S11, the detector 130 of the information processing device 1 detects that the electrode 10 is mounted on the ear, for example, in response to an operation on the operation member by the user, or the like. The processor 60a grasps that the electrode 10 is mounted on the ear on the basis of the detection signal outputted from the detector 130.

In step S12, the processor 60a causes the presentation unit 140 to present an image, a sound, or the like indicating the calibration task. Further, the processor 60a generates the timing signal S2 indicating the period of the calibration task, and outputs the timing signal S2 to the determiner 50.

In step S13, the determiner 50 performs the determination process of the mounting state of the electrode 10 (for example, the above-described process of calculating the feature quantity), with use of the measurement signal S1 and the timing signal S2. In step S14, the determiner 50 determines whether or not the mounting state of the electrode 10 on the ear is favorable.

If the determination result in step S14 is negative ("No" in step S14), the processor 60b and the presentation unit 140 perform a presentation that prompts the user to re-mount the electrode 10, and the process thereafter returns to step S12.

If the determination result in step S14 is affirmative ("Yes" in step S14), that is, if it is determined that the contact state between the ear and the electrode 10 is favorable, the information processing device 1 causes the processor 60b and the presentation unit 140 to present to the user that the mounting of the device (or the electrode 10) is completed. Thereafter, the process illustrated in the flowchart of FIG. 8 is ended.

As described above, the information processing device 1 according to the present embodiment includes the processor 60a configured to generate the timing signal S2 related to the period in which the myoelectric potential changes. Using the measurement signal S1 and the timing signal S2 enables the information processing device 1 to determine the mounting state of the electrode 10. It is possible to suppress an increase in device cost, an increase in power consumption, or the like, as compared with a case of a measurement method of checking the contact state of the electrode 10 by supplying an alternating current to the electrode 10.

In the information processing device 1 according to the present embodiment, it is possible to determine the mounting state of the electrode 10 quantitatively and relatively easily. It is possible to perform brainwave measurement while ensuring a favorable mounting state between the living body and the electrode 10. This makes it possible to enjoy a service based on highly accurate brainwave data, and to improve an availability rate of the service.

In a case where the information processing device 1 is applied to an ear electroencephalograph, it is possible to relatively easily determine the mounting state of the electrode 10 by using the myoelectric signal (myoelectric information) that is mixed into the measurement signal S1 via the electrode 10 mounted on the ear. It is possible to achieve a wearable sensor that makes it possible to suitably determine a mounting state.

The technology according to the present disclosure is applicable to a variety of products. For example, the information processing device and the measurer according to the present disclosure may be used for various electronic apparatuses with the biological measurement function. The information processing device 1 and the measurer 100 according to the present disclosure may be applied to, for example, a wearable device such as an earphone device or a headphone device.

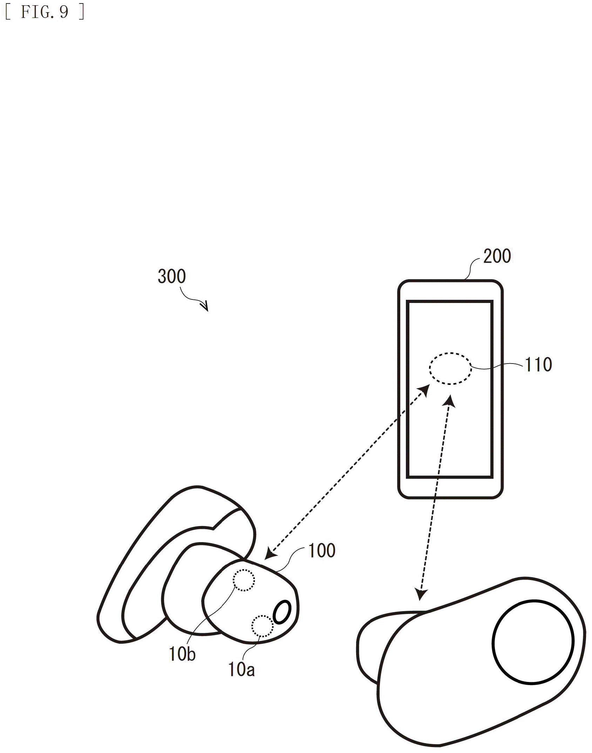

For example, the information processing device 1 may be mounted on an earphone device such as a TWS (True Wireless Stereo), as in an example illustrated in FIG. 9. One of the signal electrode 10a and the reference electrode 10b, for example, the signal electrode 10a, may be shaped as an earpiece-type dry electrode, and disposed to come into contact with an ear hole in actual use. The other of the signal electrode 10a and the reference electrode 10b, for example, the reference electrode 10b, may be disposed to come into contact with another part of the ear in actual use.

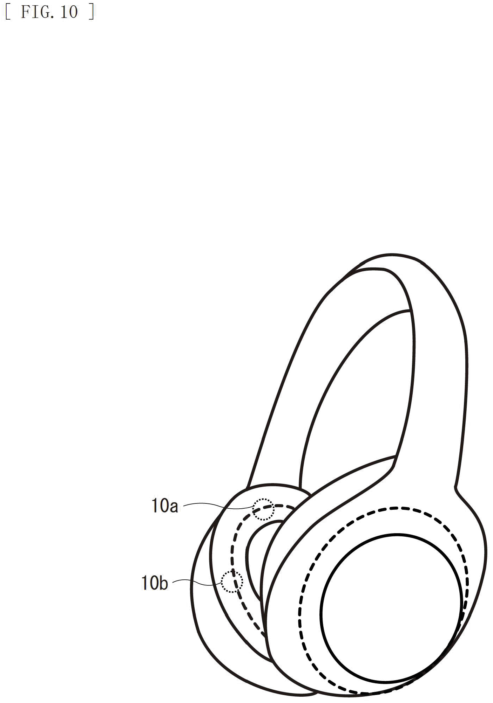

The information processing device 1 may be applied to a headphone device, as in an example illustrated in FIG. 10. For example, in a case of an overhead headphone, the signal electrode 10a and the reference electrode 10b may be mounted on a surface of an ear pad. The signal electrode 10a may be provided on one ear pad, among two ear pads, and the reference electrode 10b may be provided on the other ear pad.

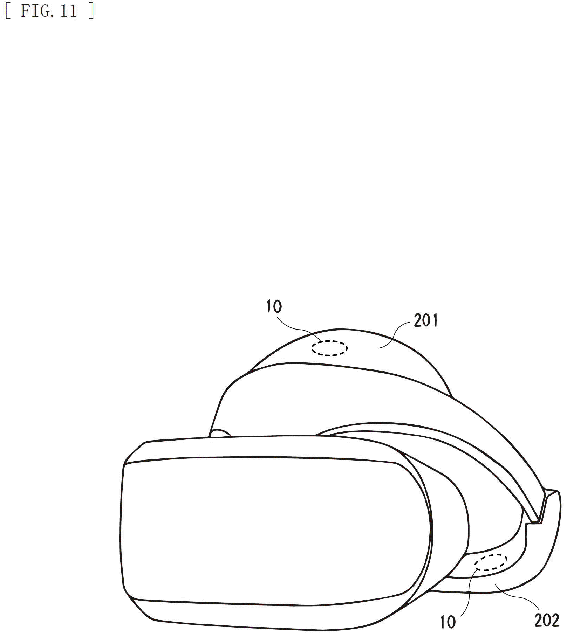

The information processing device 1 may be applied to a head-mounted display (HMD), as in an example illustrated in FIG. 11. The electrodes 10 (the signal electrode 10a, the reference electrode 10b, etc.) may be provided on a pad 201, a band 202, and the like of the head-mounted display.

The signal processor 110 and the estimator 120 (also see FIG. 1) may be implemented in the wearable device (the earphone device, the headphone device, etc.). Further, the signal processor 110 and the estimator 120 may be provided in an electronic apparatus 200, as in the example illustrated in FIG. 9. The electronic apparatus 200 is, for example, a terminal device to be used by the user. The electronic apparatus 200 may be a smartphone, a tablet terminal, a wearable terminal, a computer, or another information processing device.

As in the example illustrated in FIG. 9, the information processing device 1 may be configured as an information processing system 300 including: the measurer 100; the electronic apparatus 200 including the signal processor 110; and the like. The information processing system 300 may include, for example, the estimator 120, the display unit 141, the sound output unit 142, and the like that have been described above. It is to be noted that the signal processor 110 and the estimator 120 may be integrated with each other. The signal processor 110 may include the estimator 120.

### <Workings and Effects>

The information processing device (the information processing device 1) according to the present embodiment includes: a measurer (the measurer 100) that includes an electrode (the electrodes 10) mountable on a living body and is configured to output a first signal (the measurement signal S1) based on a potential of the electrode; a first processor (the processor 60a) configured to generate a second signal (the timing signal S2) related to a period in which a myoelectric potential of the living body changes; and a determiner (the determiner 50) configured to determine a mounting state of the electrode on the basis of the first signal and the second signal.

The information processing device 1 according to the present embodiment includes the processor 60a configured to generate the timing signal S2, and the determiner 50 configured to determine the mounting state of the electrode 10 on the basis of the measurement signal S1 and the timing signal S2. Accordingly, it is possible to achieve an information processing device that makes it possible to suitably determine a mounting state.

Next, description is given of modification examples of the present disclosure. Hereinafter, components similar to those in the foregoing embodiment are denoted by the same reference numerals, and descriptions thereof are omitted as appropriate.

### <2. Modification Examples>

Although a configuration example of the information processing device 1 has been described in the above-described embodiment, the configuration is merely an example, and the configuration of the information processing device 1 is not limited to the above-described example. Further, although an example of a method of determining the mounting state has been described above, the method of determining the mounting state is not limited to the above-described example.

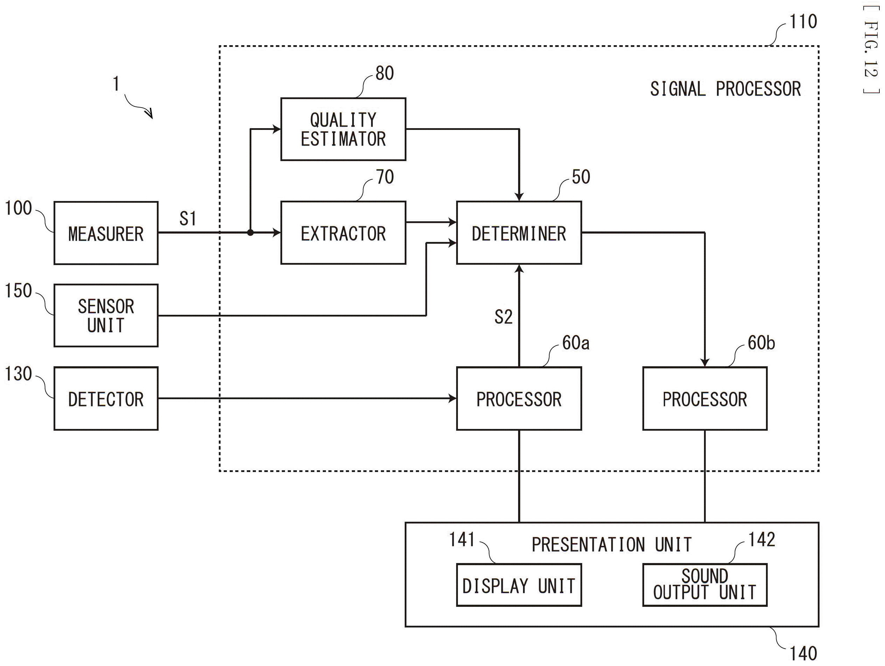

FIG. 12 is a diagram for describing a configuration example of an information processing device according to a modification example of the present disclosure. As in the example illustrated in FIG. 12, the information processing device 1 may include an extractor 70 and a quality estimator 80. For example, the signal processor 110 includes the extractor 70 and the quality estimator 80. The extractor 70 and the quality estimator 80 may include a logic circuit (a digital circuit) configured to perform various kinds of digital signal processing.

The extractor 70 is configured to extract a myoelectric potential component. The extractor 70 may, for example, extract the myoelectric component included in the measurement signal S1 by a signal separation technique, and output the measurement signal S1 indicating the myoelectric component to the determiner 50. The determiner 50 may perform the determination process of the mounting state with use of the measurement signal S1 from which unnecessary signal components have been removed, that is, the extracted myoelectric component.

The extractor 70 may use, as the signal separation technique, a technique described in Non-Patent Literature (Chen, Xun, et al. "A novel EEMD-CCA approach to removing muscle artifacts for pervasive EEG." IEEE Sensors Journal 19.19 (2018): 8420-8431). It is possible to determine the mounting state with high accuracy by removing potential signals other than the myoelectric signal, such as a potential change caused by cerebral activity, by a signal separation process.

The determiner 50 may use, as the feature quantity to be used in the determination process of the mounting state, an integrated intensity of the measurement signal S1 in a high frequency band (e.g., 20 Hz or higher) in which a frequency response of the myoelectric signal is strongly reflected. The determiner 50 may determine whether the mounting state of the electrode 10 is favorable or poor by, for example, comparing the integrated intensity of the measurement signal S1 with a predetermined threshold.

Depending on a kind of the calibration task, the myoelectric signal can be generated in only a small number of muscle fibers, and frequencies at which a signal intensity becomes strong during tensing can decrease. Hence, the determiner 50 may use, as the feature quantity, the integrated intensity in a frequency band in which the signal intensity of a predetermined value or greater is exhibited.

The determiner 50 may individually calculate the feature quantity during tensing and the feature quantity during relaxing by using the timings of tensing and relaxing presented in the calibration task, and use the calculated values as the feature quantity. Further, the determiner 50 may determine the mounting state by comparing the feature quantity distributions during tensing and during relaxing with each other.

The information processing device 1 may include the quality estimator 80 as in the example illustrated in FIG. 12. The quality estimator 80 is configured to estimate a signal quality of the measurement signal S1. The quality estimator 80 may, for example, estimate the signal quality of the measurement signal S1, and output an output signal indicating the signal quality of the measurement signal S1 to the determiner 50. The quality estimator 80 may output, to the determiner 50, the output signal indicating, for example, a period in which the signal quality of the measurement signal S1 is estimated to be relatively favorable, during the period of the action involving a change in the myoelectric potential.

The determiner 50 may determine the mounting state with use of the measurement signal S1 in the period in which the signal quality is relatively favorable, on the basis of the output signal of the quality estimator 80. It is possible to perform the determination process of the mounting state in consideration of the quality of the measurement signal S1, which makes it possible to perform the determination process of the mounting state with high accuracy.

The information processing device 1 may include a sensor unit 150 as illustrated in FIG. 12. The sensor unit 150 includes a sensor configured to detect the contact (or proximity) of the electrode 10 with the living body. Examples of the sensor include an acceleration sensor, an optical sensor, an ultrasonic sensor, a temperature sensor, and a microphone. The sensor unit 150 may also include a sensor configured to measure a contact impedance between the living body and the electrode 10.

The determiner 50 may determine whether the contact state of the electrode 10 is favorable or poor on the basis of the measurement signal S1 and the output signal of the sensor unit 150. In this case, it is possible to perform the determination process of the mounting state in consideration of a result of the detection by the sensor unit 150, which makes it possible to accurately perform the determination process of the mounting state.

The determiner 50 may perform the determination process of the mounting state by classifying the kurtosis of the feature quantity distribution by using machine learning (a learning model). It is to be noted that a technique of the machine learning is not particularly limited, and various techniques may be used. The kurtosis of the feature quantity distribution, the feature quantity, the measurement signal S1, or the like may be used as an input signal in a case of performing the machine learning.

The determiner 50 of the information processing device 1 may calculate the feature quantity distribution during the calibration task, and determine whether or not a change in the myoelectric signal sufficient for the determination of the mounting state has been measured. If a sufficient change in the myoelectric signal has not been measured, a dynamic presentation may be performed that continues to present tensing and relaxing of the muscle until the mounting state is determined to be favorable.

If it is estimated by the quality estimator 80 that the signal quality of the measurement signal S1 during the calibration task is poor, the information processing device 1 may prompt the user to re-mount the electrode 10. Further, the information processing device 1 may determine whether or not the calibration task is appropriately performed by the user with use of the output signal of the sensor unit 150. The information processing device 1 may determine whether or not the calibration task is appropriately performed with use of an image captured by an external camera.

The calibration task may be started in response to an operation (e.g., a button operation) on the operation member by the user, or the calibration task may be started in response to attachment or detachment of the device from a charging case. Further, the calibration task may be started in a case where the proximity of the device to the living body is detected by the sensor unit 150.

The information processing device 1 may decide to start the calibration task if it is detected that the mounting state has become poor on the basis of the output signal of the quality estimator 80, the output signal of the sensor unit 150, or the like. It is to be noted that the calibration task is not limited to the above-described example. The action of tensing the muscle may be, for example, an action of pursing lips, an action of tilting the head, or the like. Further, the action of relaxing the muscle may be, for example, resting, loosening up, half-opening the mouth, or the like.

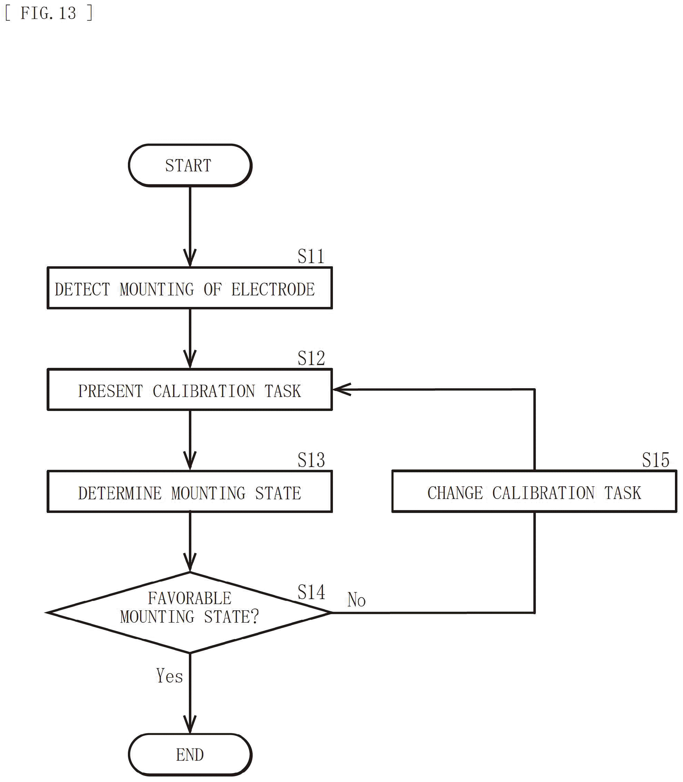

The information processing device 1 may change what is to be performed as the calibration task, on the basis of the result of the determination by the determiner 50. For example, as illustrated in FIG. 13, if the determination result in step S14 is negative ("No" in step S14), the calibration task may be changed in step S15, and the changed calibration task may be presented to the user in step S12.

Although the description has been given hereinabove of the present disclosure with reference to the embodiments and modification examples, the present technology is not limited to the foregoing embodiments and the like, and may be modified in a wide variety of ways. For example, although the above-described modification examples have been described as the modification examples of the above-described embodiments, the configurations of the respective modification examples may be combined as appropriate. In addition, the present disclosure has applicability not only to the human body, but also to a living body other than the human body, for example, animals such as pets and domestic animals.

An information processing device according to an embodiment of the present disclosure includes: a measurer that includes an electrode mountable on a living body and is configured to output a first signal based on a potential of the electrode; a first processor configured to generate a second signal related to a period in which a myoelectric potential of the living body changes; and a determiner configured to determine a mounting state of the electrode on the basis of the first signal and the second signal. Accordingly, it is possible to achieve an information processing device that makes it possible to suitably determine a mounting state.

An information processing system according to an embodiment of the present disclosure includes: a measurer that includes an electrode mountable on a living body and is configured to output a first signal based on a potential of the electrode; and an information processing device including a first processor configured to generate a second signal related to a period in which a myoelectric potential of the living body changes, and a determiner configured to determine a mounting state of the electrode on the basis of the first signal and the second signal. Accordingly, it is possible to achieve an information processing system that makes it possible to suitably determine a mounting state.

It is to be noted that the effects described in the present specification are merely examples and are not limited to the description, and other effects may be obtained. Further, the present disclosure may have the following configuration.
(1) An information processing device including:
   a measurer that includes an electrode mountable on a living body and is configured to output a first signal based on a potential of the electrode;
   a first processor configured to generate a second signal related to a period in which a myoelectric potential of the living body changes; and
   a determiner configured to determine a mounting state of the electrode on the basis of the first signal and the second signal.
(2) The information processing device according to (1), in which the first processor is configured to execute a process of prompting a first action involving a change in the myoelectric potential.
(3) The information processing device according to (2), in which the first processor is configured to generate, as the second signal, a signal related to a period of the first action.
(4) The information processing device according to (2) or (3), in which the first processor is configured to generate, as the second signal, a signal related to a start timing and an end timing of the first action.
(5) The information processing device according to any one of (2) to (4), in which the determiner is configured to determine the mounting state of the electrode on the basis of the first signal in a period of the first action.
(6) The information processing device according to any one of (2) to (5), in which the first processor is configured to execute a process of prompting the first action of performing tensing and relaxing of a muscle of a face or a neck.
(7) The information processing device according to any one of (2) to (6), in which the first processor is configured to execute a process of prompting the first action of repeating tensing and relaxing of a muscle of a face or a neck.
(8) The information processing device according to any one of (2) to (7), in which the first processor is configured to execute a process of presenting an image, a sound, or both prompting the first action.
(9) The information processing device according to any one of (1) to (8), further including a second processor configured to execute a process of presenting an image, a sound, or both based on a result of the determination by the determiner.
(10) The information processing device according to any one of (1) to (9), in which the determiner is configured to determine the mounting state of the electrode on the basis of an amplitude of the first signal in the period in which the myoelectric potential of the living body changes.
(11) The information processing device according to any one of (1) to (10), in which the determiner is configured to determine the mounting state of the electrode on the basis of a distribution of values of the first signal in the period in which the myoelectric potential of the living body changes.
(12) The information processing device according to any one of (1) to (11), further including a detector configured to detect mounting of the electrode on the living body.
(13) The information processing device according to (12), in which the first processor is configured to execute a process of prompting a first action involving a change in the myoelectric potential in a case where the mounting of the electrode is detected by the detector.
(14) The information processing device according to any one of (1) to (13), in which
   the electrode is configured to be in contact with an ear or a region around the ear, and
   the measurer is configured to output the first signal based on the myoelectric potential.
(15) An information processing system including:
   a measurer that includes an electrode mountable on a living body and is configured to output a first signal based on a potential of the electrode; and
   an information processing device including
      a first processor configured to generate a second signal related to a period in which a myoelectric potential of the living body changes, and
      a determiner configured to determine a mounting state of the electrode on the basis of the first signal and the second signal.
(16) The information processing system according to (15), in which the first processor is configured to execute a process of prompting a first action involving a change in the myoelectric potential.
(17) The information processing system according to (16), in which the first processor is configured to generate, as the second signal, a signal related to a period of the first action.

This application claims the benefit of Japanese Priority Patent Application JP2023-194584 filed with the Japan Patent Office on November 15, 2023, the entire contents of which are incorporated herein by reference.

It should be understood by those skilled in the art that various modifications, combinations, sub-combinations, and alterations may occur depending on design requirements and other factors insofar as they are within the scope of the appended claims or the equivalents thereof.

## Claims

1. An information processing device comprising:
a measurer that includes an electrode mountable on a living body and is configured to output a first signal based on a potential of the electrode;
a first processor configured to generate a second signal related to a period in which a myoelectric potential of the living body changes; and
a determiner configured to determine a mounting state of the electrode on a basis of the first signal and the second signal.

2. The information processing device according to claim 1, wherein the first processor is configured to execute a process of prompting a first action involving a change in the myoelectric potential.

3. The information processing device according to claim 2, wherein the first processor is configured to generate, as the second signal, a signal related to a period of the first action.

4. The information processing device according to claim 2, wherein the first processor is configured to generate, as the second signal, a signal related to a start timing and an end timing of the first action.

5. The information processing device according to claim 2, wherein the determiner is configured to determine the mounting state of the electrode on a basis of the first signal in a period of the first action.

6. The information processing device according to claim 2, wherein the first processor is configured to execute a process of prompting the first action of performing tensing and relaxing of a muscle of a face or a neck.

7. The information processing device according to claim 2, wherein the first processor is configured to execute a process of prompting the first action of repeating tensing and relaxing of a muscle of a face or a neck.

8. The information processing device according to claim 2, wherein the first processor is configured to execute a process of presenting an image, a sound, or both prompting the first action.

9. The information processing device according to claim 1, further comprising a second processor configured to execute a process of presenting an image, a sound, or both based on a result of the determination by the determiner.

10. The information processing device according to claim 1, wherein the determiner is configured to determine the mounting state of the electrode on a basis of an amplitude of the first signal in the period in which the myoelectric potential of the living body changes.

11. The information processing device according to claim 1, wherein the determiner is configured to determine the mounting state of the electrode on a basis of a distribution of values of the first signal in the period in which the myoelectric potential of the living body changes.

12. The information processing device according to claim 1, further comprising a detector configured to detect mounting of the electrode on the living body.

13. The information processing device according to claim 12, wherein the first processor is configured to execute a process of prompting a first action involving a change in the myoelectric potential in a case where the mounting of the electrode is detected by the detector.

14. The information processing device according to claim 1, wherein
the electrode is configured to be in contact with an ear or a region around the ear, and
the measurer is configured to output the first signal based on the myoelectric potential.

15. An information processing system comprising:
a measurer that includes an electrode mountable on a living body and is configured to output a first signal based on a potential of the electrode; and
an information processing device including
a first processor configured to generate a second signal related to a period in which a myoelectric potential of the living body changes, and
a determiner configured to determine a mounting state of the electrode on a basis of the first signal and the second signal.

16. The information processing system according to claim 15, wherein the first processor is configured to execute a process of prompting a first action involving a change in the myoelectric potential.

17. The information processing system according to claim 16, wherein the first processor is configured to generate, as the second signal, a signal related to a period of the first action.
